# EUROPEAN PATENT SPECIFICATION

(11) **EP 1 319 926 B1**
(45) Date of publication and mention of the grant of the patent: **14.05.2014**
(21) Application number: 02466003.7
(22) Date of filing: 28.11.2002
(51) Int. Cl.: G01B 11/10, G01N 33/36, D01H 13/32

(54) **A device for contactless measurement of the diameter of a moving yarn or a yarn-like textile formation**
Vorrichtung zur kontaktlosen Messung des Durchmessers eines sich bewegenden Garnes oder einer garnähnlichen Textilformation
Dispositif pour mesurer sans contact le diamètre d'un fil en mouvement ou d'une formation du type fil en mouvement

(30) Priority: 17.12.2001 CZ 20014561
(43) Date of publication of application: 18.06.2003
(73) Proprietor: Rieter CZ a.s., 562 15 Usti nad Orlici (CZ)
(72) Inventor: Sloupensky, Jiri, 562 01 usti nad Orlici (CZ); Stusak, Miroslav, 561 12 Brandys nad Orlici (CZ); Kousalik, Pavel, 562 01 Usti nad Orlici (CZ); Hajek, Ladislav, 561 12 Brandys nad Orlici (CZ)
(74) Representative: Musil, Dobroslav

(56) References cited:
- WO-A-00/28285
- US-A- 5 499 794
- US-B1- 6 219 135

## Description

### Technical field

The invention relates to a device for contactless measurement of diameter of a moving yarn or of a yarn-like textile formation comprising an optical line sensor with a plurality of pixels arranged next to each other in at least one row, which is integrated into one semiconductor integrated custom ASIC circuit together with a control module for timing pulse rate generation and a comparator for processing and/or evaluating of line optical sensor signal.

### Background of the invention

Well-known devices for measuring moving yarn on a spinning or winding machine comprise a measuring head with a sensor of its own, an evaluation unit, and a control unit which are mutually interconnected by lines.

Such an arrangement usually requires much space while a spinning or winding machine on the contrary requires the device to take up only a minimum space along the yarn path. For this reason, the measuring head is carried out as simple as possible, adapted only for measuring and processing the measuring signal while the evaluation and other operations are carried out in an evaluating unit and/or in a control unit. Consequently, the device has a considerable number of components interconnected by many lines and connectors which increases both its acquisition costs and the complexity of its installation on the machine.

The drawbacks of this solution have been removed by the EP 0945533 A1 describing a device in which a measuring cell is connected to a processor related exclusively to said measuring cell. The measuring cell and the processor are mounted on a common holder and seated in one case. The processor has a digital output which results in a limitation of the obtainable reliability.

This solution is intended in particular for measuring cells emitting an analog signal but it can contain also sensors emitting digital signals such as optical line CCD sensors transmitting into the processor in digital form information on which of the sensor pixels is illuminated and which is not.

In view of the number of sensor pixels required for sufficiently exact measuring of the yarn diameter and of the number of measurements per time unit, this arrangement requires high data throughput between the sensor and the processor which places heavy demands on the connected processor and in general limits the evaluating possibilities of the device. For instance for 1,000 pixels 1,000 information bits are transmitted at each measurement.

A well-known device uses for the reading of the state of the sensor pixels the processor input with a maximum frequency of the processor which is nowadays usually about 2 Mhz. This limits the number of measurements per time unit and thus reduces the measurement quality, in particular for high delivery speeds, or permits only the use of a sensor with a lower number of pixels.

The US 5.499.794 describes a device for contactless measurement of properties of a moving yarn or of a yarn-like textile formation comprising a line optical sensor integrated into one semiconductor integrated custom ASIC circuit together with a circuit for timing pulse rate creation, an amplifier and a comparator for processing and/or evaluating the signal of the line optical sensor.

The US 6.219.135 B1 describes a device for an optical recording of at least one parameter of a yarn material moving in a longitudinal direction comprising an optical sensor composed of at least two individual sensors, where at least one individual sensor is constructed and positioned so that at least one measured value is digitally recorded/sensed for a parameter and one individual sensor is fitted with analog recording/sensing of measured value for a parameter. The device consists of two identically created circuits, which can be assigned to each individual sensor. Each of them consists of a member for converting the light into electric current, for instance a photodiode. The mentioned element is advantageously considered as an individual sensor itself.

Further it is connected in series with other members for conversion of its output signal, such as an amplifier, a comparator and a memory. The amplifier, which consists of a computing amplifier and a condensator (in the feedback part) is further connected in parallel with a circuit closer. The memory device is connected with the multiplexer output. With the multiplexer is cross-connected in series an evaluating circuit, which can be advantageously created as a computing element. In addition to that is to the evaluating circuit optionally connected a circuit for analog individual signal generation. The individual sensor further consists of an operational amplifier with a capacitor and a resistor in parallel. Also this Patent comprises a reference to integration of at least a part of above mentioned members with individual sensor into a form of an integrated circuit. However it does not specify, which members are supposed to be integrated with which individual sensor and in what manner. Cutting down on demands on data transmission efficiency between the sensor and the evaluation device is solved by a use of two sensors.

The invention intends to limit or to eliminate the drawbacks of the background art and to reduce the demands placed on the data throughput between the optical sensor comprising a system of pixels and the evaluating device or the circuits for processing the sensor signals in consideration of the specific properties of the output signal of the optical line sensor in measuring a yarn or a yarn-like textile formation.

### Principle of the invention

The goal of the invention has been reached by a device for contactless measurement of diameter of a moving yarn or a yarn-like formation as defined in claim 1, which principle consists in that the semiconductor integrated custom ASIC circuit further comprises a counter of shadowed pixels connected via a gate towards the output of the comparator, to which output is connected a module of a data bus connectable to a data bus for communication with a control and/or evaluation processor.

The advantage of this arrangement consists in particular in that the initial operations of the processing and/or evaluating of the sensor signal take place in one integrated circuit so that they are not limited by the capacity of the data flow between the sensor and the processor because there is no need to transmit from the output of the device detailed information on which pixel is/is not illuminated. Instead, transmitted directly in the digital form is the data on the yarn diameter placing substantially reduced demands on the data throughput because for instance for 1,000 pixels instead of 1,000 information bits only 10 information bits on the yarn width or on the number of continually shadowed and/or irradiated pixels will do. The circuits for processing and/or evaluating the signal integrated with the sensor are able to operate on the same frequency as the sensor, which in the optical line sensors CMOS nowadays used currently lies between 20 an 40 Mhz.

Another advantage of the solution according to the invention consists in the reduction of the costs of acquisition of the device due to the fact that a part of the electronic circuits for processing the signal of the sensor is integrated together with the sensor in one case of the integrated circuit or, as the case may be, directly on a common semiconductor die which substantially reduces the need of using external electronic circuits between the sensor and the processor.

From the technological reasons it is sometimes suitable to know the position of the yarn in front of the line optical sensor. To reach this effect electronic circuits for processing and/or evaluating the signal of the line optical sensor, which are integrated with an line optical sensor contain means for determining and evaluating the yarn position in front of the line optical sensor, while on the comparator output is connected the shadow edge detector, whose output is connected to the auxiliary counter input, which is concurrently connected with the timing output and the resetting output of the control module for determining the position of the beginning of the yarn shadow on the optical sensor, while the auxiliary counter output is connected with the module of the data bus input.

For monitoring the yarn diameter is between the counter of shadowed pixels and the module of the data bus inserted a divider, while between the divider and the control module (305) is inserted a counter of the number of measurement cycles and between divider, control module and the counter (310) of a number of measurement cycles is inserted a decision taking module, while resetting output of the control module is connected to the resetting input of the counter of the shadowed pixels and to the resetting input of a counter of the number of the measurement cycles.

In another variant of the device, the decision taking module contains information about a constant number of measurement cycles for determining the mean value of yarn diameter from a constant number of measurements, which means that the monitored yarn section is constant in length or in time.

In another variant of the device, the decision taking module comprises means for determining a variable number of measurement cycles for determining the mean value of the yarn diameter from a variable number of measurements and thus permits to change in the course of the measurement process the length or the time of the monitored yarn section.

Another variant of the device permits continuous evaluation of various length or time sections of the monitored yarn in which the first measuring cycle provides the mean value of the yarn diameter which is after each completed cycle recalculated and is ready to be read. For this reason, between the counter of shadowed pixels and the module of the data bus is inserted a divider, while between the divider and the control module is inserted a counter of a number of measurement cycles, while outputs of the control module are interconnected with the counter of the shadowed pixels, with the divider and with the counter of the number of the measurement cycles, while outputs of the counter of the number of the measurement cycles are interconnected with the divider.

To eliminate the risk of interpreting as yarn shadow soiled parts of the sensor counter of shadowed pixels is created as a circuit constructed by to the output of the gate connected switch (3031) and the detector (3036) of shadow edge, whose output is connected with the input of the switch (3031), while to the outputs of the switch (3031) is connected an optional number of input counters (3032a, 3032b, ... 3032n), from which one is connected to the module for the selection of the maximum and the others are connected to the module (3033) for the selection of the maximum and at the same time to the detector (3034) of plurality of shadows, while the module (3033) for the selection of the maximum output is connected with the divider (309) input, a terminal counter (3035), the module (3033) for the selection of the maximum, the detector (3034) of plurality of shadows and the input counters (3032a, 3032b, ... 3032n) are connected with the resetting output of the control module (305) and the detector (3034) of plurality of shadows is fitted with an output connected to the module (304) of the data bus for monitoring of more than one shadow during one measurement cycle and selection of at least maximal shadow. The evaluating circuit monitoring the yarn diameter comprises means for monitoring more than one shadow during one measurement cycle and means for the selection of at least one maximum shadow which is expected to be the shadow of the yarn.

The device according to the invention simplifies the installation of the device for contactless measurement of the properties of a moving yarn or of a yarn-like textile formation and improves the quality properties of the optical line sensor, in particular by eliminating the problems connected with the data transfer between the sensor and the electronic circuits for processing and/or evaluating the signal of the optical line sensor.

### Description of the drawings

Examples of embodiment of the device according to the invention are schematically shown in the enclosed drawings in which Fig. 1 is an integrated ASIC custom circuit, Fig. 2a an example of embodiment of electronic circuits for processing and/or evaluating the signal of the optical line sensor for determining the yarn diameter in each measurement cycle, Fig. 2b the device shown in Fig. 2a completed by a device for detecting and evaluating the yarn position in front of the optical line sensor, Fig. 3 is an example of embodiment of electronic circuits for processing and/or evaluating the signal of the optical line sensor for detecting the value of the yarn diameter in each measurement cycle, Figs. 4 and 5 examples of embodiment of electronic circuits for processing and/or evaluating the signal of the optical line sensor for determining the mean value of the yarn, and Fig. 6 an example of embodiment of electronic circuits in which the evaluating circuit comprises means for monitoring more than one shadow during one measurement cycle and for the selection of at least the maximum shadow.

### Examples of embodiment

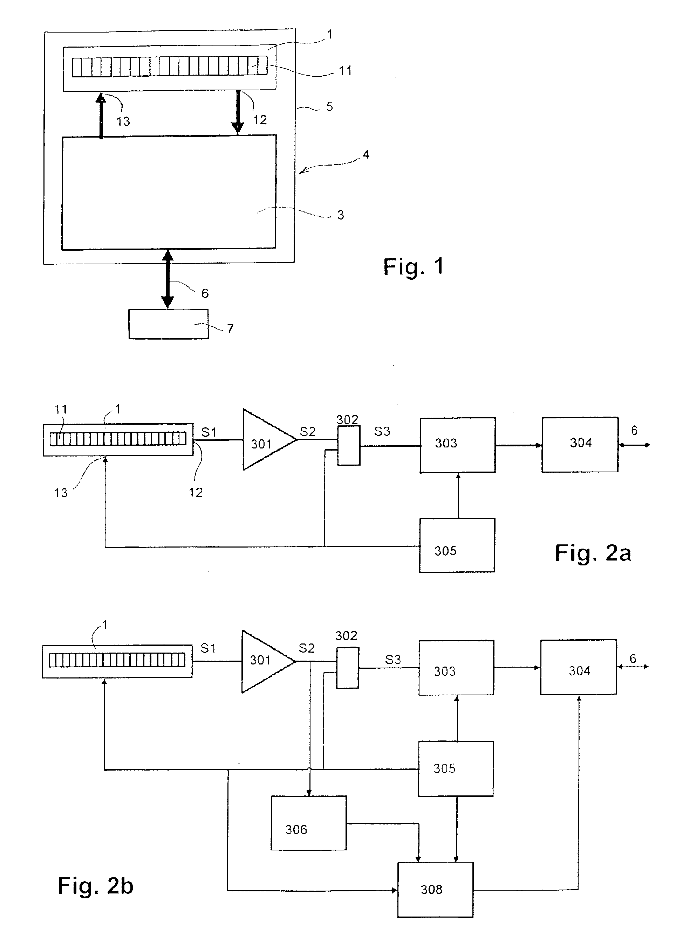

The device for contactless measurement of at least one property of a moving yarn or of a yarn-like textile formation comprises an optical line sensor **1** having in at least one row next to each other situated pixels **11** which are radiation-sensitive sensing elements. Nowadays are currently available single-row optical sensors, but it is possible in case of need to arrange a number of single-row sensors in a configuration, or to build a custom ASIC circuit with a many-row optical sensor. This optical line sensor **1** is made on a semiconductor die which contains also circuits **3** for processing and/or evaluating the signals of the optical line sensor **1** with which the optical line sensor **1** is integrated into one semiconductor integrated custom ASIC circuit **4.** The semiconductor integrated custom ASIC circuit **4**. is in a well-known manner seated in a case **5** shown in Fig. 1 only simplified by a circumference line.

Within the system of mutual interconnection, the optical line sensor **1** is coupled with electronic circuits **3** for processing and/or evaluating the signal of the optical line sensor **1** at least on the output **12** of the sensor which is used for information transfer from the sensor **1** into the electronic circuits **3** for processing and/or evaluating the signals of the sensor **1,** and on the input **13** of the sensor used for controlling the sensor **1** by said electronic circuits **3** for processing and/or evaluating the signals of the sensor **1**. The input **13** and the input **12** of the sensor 1 are also intended to mean the system of inputs and outputs the sensor **1** can be fitted with.

The electronic circuits **3** for processing and/or evaluating the signals of the optical line sensor **1**, integrated with sensor **1**, are by means of a two-way data bus **6** connected with a processor **7** used for evaluating, monitoring and controlling the electronic circuits **3** and by means of them also the optical line sensor **1**.

An example of embodiment of the electronic circuits **3** integrated with the sensor **1** on a common semiconductor die is shown in Fig. 2a where to the output of the sensor **1** there is connected the input of a comparator **301** whose output is via a gate **302** connected with a counter **303** of shadowed pixels **11** of the sensor. The output of the counter **303** of the shadowed pixels is connected to a module **304** of a data bus whose output or, as the case may be, outputs and inputs are connectable to the well-known data bus **6**. The device also comprises a control module **305** whose outputs are connected with the counter **303,** with the gate **302,** and with the optical line sensor **1**. The module **304** of the data bus is common with the communication module for the communication with the control and/or evaluation processor **7**.

This example of embodiment is intended for the simple measurement of a yarn property such as the yarn diameter where the clock frequency coming from the control module into the sensor **1** supplies in one measurement cycle information on irradiation (illumination), shadowing or partial shadowing of all the pixels **11** of the sensor **1** which is led from the output of the sensor as an analog signal **S1** into the comparator **301** where it is transformed into a signal **S2** indicating the dimension of the shadow produced by the yarn on the sensor **1** and led into the gate **302** in which it is keyed by the clock rate of the control module **305**. On the output of the gate **302** there is generated a signal **S3** whose number of pulses corresponds to the number of the shadowed pixels of the sensor **1**. The signal **S3** indicating the number of the shadowed pixels is led into the counter **303** of the shadowed pixels in which is counted the number of pulses in one measurement cycle, i.e., the number of the pixels **11** of the sensor **1** which are shadowed by the yarn. The information on the number of the shadowed pixels **11** of the sensor is led into the module **304** of the data bus where it is transformed in a form suitable for the transfer by the data bus **6** for the further processing in the processor **7**. The control module **305** creates a signal indicating the end of the measurement cycle on whose basis the counter **303** of the shadowed pixels of the sensor is set at zero and thus prepared for starting the counting of the shadowed pixels **11** of the sensor in the subsequent measurement cycle.

In some technological cases, it is advantageous, in monitoring the yarn properties, to obtain additional information on the yarn position in front of the optical line sensor **1**. For this reason, the device shown in Fig. 2a is improved as shown in Fig. 2b. The improved device contains an auxiliary counter **308** used to detect the position of the beginning of the yarn shadow on the optical sensor **1**. The auxiliary counter **308** receives from the control module **305** clock pulses and zero-setting signal **N** indicating the beginning of the counting of the sensor **1**. The device also contains a detector **306** of the shadow edge which is connected to the output of the comparator **301**. The signal on the start of the signal **S2**, i.e., on the transition of the irradiation state of the pixels **11** of the sensor **1** from the irradiated to the shadowed state, is led from the detector **306** of the shadow edge into the auxiliary counter **308** and stops there the counting carried out by the auxiliary counter **308.** The auxiliary counter **308** is thus reset and re-started at the beginning of each measurement cycle and counts the number of the illuminated (irradiated) pixels **11** of the sensor as long as up to the detection of the beginning of the shadow by the detector **306** of the shadow edge. The information on the position of the yarn in the sensor **1** is led into the module **304** of the data communication where it is processed into a signal suitable to be led by the data bus **6** into the processor **7**.

Each component of the example of embodiment shown in Fig. 2b can in case of need operate independently, i.e., to measure the yarn diameter and/or to monitor the position of the yarn or, more exactly, of its shadow in front of the optical line sensor **1**. Besides this, the embodiment shown in Fig. 2b can be used also in the other below described embodiments, if the information on the yarn position in front of the optical line sensor **1** is required for technological or for other reasons.

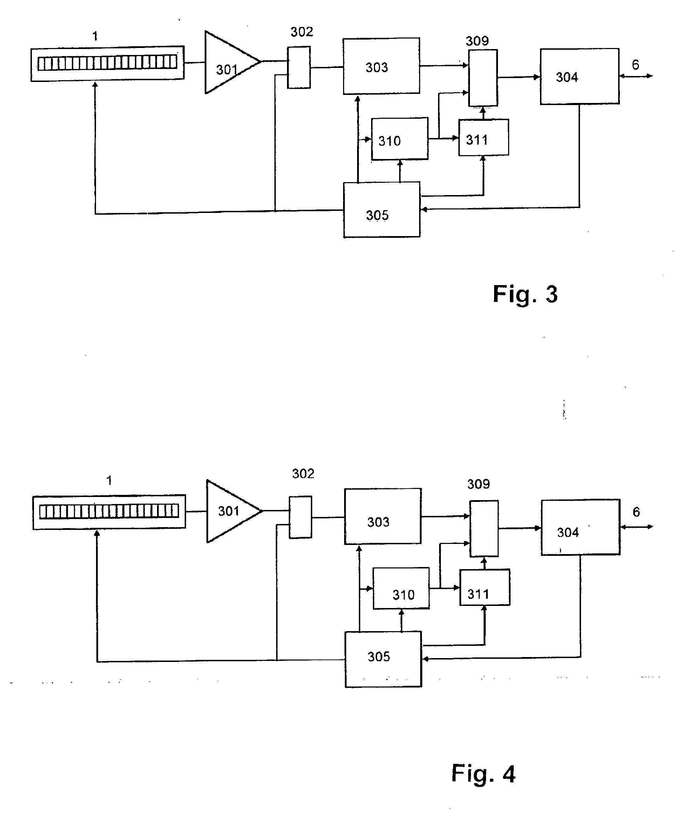

In another example of embodiment shown in Fig. 3, the system of electronic circuits of the Fig. 2 is supplemented by a divider **309** situated between the counter **303** of the shadowed pixels **11** of the sensor **1** and the module **304** of the data bus **6**.

The zero-setting (resetting) output of the control module **305** is connected to the resetting input of the counter **303** of the shadowed pixels of the sensor **1** and to the resetting input of a counter **310** of the number of the measurement cycles which is interconnected between the control module **305** and the divider **309.** Between the control module **305,** the counter **310** of the number of the measurement cycles, and the divider **309** there is interconnected also a decision taking module **311** with a constant set number **N** of the measurement cycles which states whether the **N** measurement cycles already have been completed and consequently, in positive case, that the values of the counters **303** and **310** are valid.

In this solution, the counter **303** of the shadowed pixels **11** of the sensor **1** counts the sum of the shadowed pixels **11** of the sensor **1** from the measurement cycles effected after the resetting of the counter **303** of the shadowed pixels **11** which provides information on the number **X** of the shadowed pixels **11** of the sensor **1** which is led into the divider **309.** At the same time, the counter **310** of the number of the measurement cycles monitors the number of the cycles effected since the last resetting and as soon as this number reaches the value **N** stored in the decision taking module **311** the counter **310** of the number of the measurement cycles sends a signal to the divider **309** in which the number **X** of the shadowed pixels **11** is divided by the preset number **N** of the measurement cycles. On the output of the divider **309** is then generated a signal giving the information on the mean value of the number of the shadowed pixels **11** of the sensor **1**. After the processing in the module **304** of the data bus, information is obtained on the mean value of the yarn diameter in the time or yarn length corresponding to the preset number **N** of the measurement cycles and to the yarn delivery speed.

In the described embodiment, the divider **309** fulfils the function of the arithmetic mean of the number **X** of the shadowed pixels **11** of the sensor **1** out of the number **N** of the measurement cycles. In another example of embodiment, the divider **309** is fitted with another function for determining the mean value of the number **X** of the shadowed pixels **11** of the sensor **1** out of the number **N** of the measurement cycles such as a gliding mean value, etc.

The number **N** of the measurement cycles undergoes no change during the production of a given yarn lot; it changes for instance when starting a new lot with different parameters such as the fineness of the yarn to be produced, its composition, and/or its delivery speed. In such a case, the change is carried out by inserting a new number **N** of the measurement cycles from the control module **305** into the decision taking module **311**.

In a not shown embodiment, the number **N** of the measurement cycles is inserted directly into the decision taking module **311**.

Fig. 4 shows an embodiment of the device with adjustable division ratio of the number **X** of the shadowed pixels **11** of the sensor **1** and of the variable number **Y** of the measurement cycles. The number **Y** of the measurement cycles is governed by the control module **305**, for instance according to the variable delivery speed in the course of spinning.

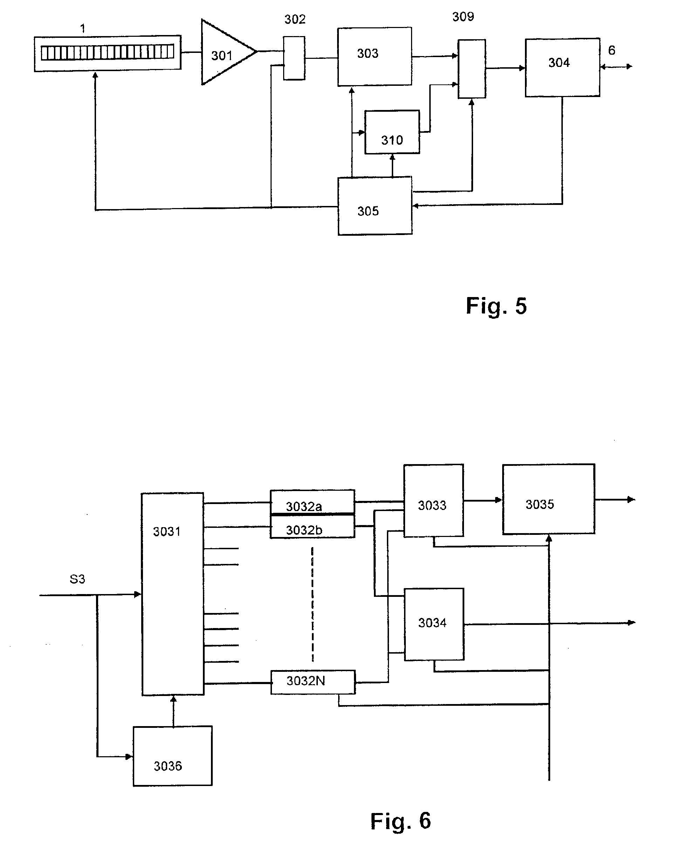

In another example of embodiment, shown in Fig. 5, the configuration of the circuits is analogous to the embodiment shown in Fig. 3 or 4, but the decision taking module **311** is omitted. The outputs of the control module **305** are interconnected with the divider **309** and with the counter **310** of the number of the measurement cycles. The outputs of the counter **310** of the number of the measurement cycles are interconnected with the divider **309**.

The counter **303** of the shadowed pixels of the sensor sums up the number of the shadowed pixels **11** of the sensor counted in the measurement cycles carried out since the resetting of the counter **303** of the shadowed pixels and thus produces information on the number **X** of the shadowed pixels **11** of the sensor which is led into the divider **309**. The divider **309** simultaneously receives information on the number **Y** of the effected measurement cycles from the counter **310** of the number of the measurement cycles. The information on the stop of the running measurement cycle is supplied to the counter **310** of the number of the measurement cycles from the control module **305.** The number **X** of the shadowed pixels **11** of the sensor **1** is in the divider **309** continuously divided by the number **Y** of the measurement cycles just carried out. On the output of the divider **309** is then generated a signal containing information on the topical mean value of the number of the shadowed pixels **11** of the sensor **1** which is on the output of the divider **309** available for reception by the module **304** of the data bus where it is transformed into information on the mean value of the yarn diameter in a length or time interval corresponding to the delivery speed of the yarn and to the number **Y** of the measurement cycles out of which the value of the mean number of the shadowed pixels has been stated. This value is available for reading on the data bus **6**, for instance by means of the processor **7**. After the reading of the topical information on the mean value of the yarn diameter from the module **304** of the data bus, the module **304** sends information on the carried out reading into the control module **305** which sends out instruction to reset the counter **303** of the shadowed pixels, (to reset) the counter **310** of the number of the measurement cycles **309**, and to reset the divider **309**, thus rendering these circuits ready for further operation.

This method permits to obtain information on the mean value of the yarn diameter at various length sections of the yarn according to the topical requirements of the processor or of the control unit of the operating unit and/or of a machine section and/or of the machine.

The electronic circuits **3** for processing and/or evaluating the signal of the optical line sensor integrated with the optical line sensor **1** are given only by way of example and can be replaced by another connection which will fulfil the specific demands.

The above examples of embodiment do not solve the problem if the pixels are shadowed interruptedly, for instance by the yarn and by impurity particles such as dust or a piece of a fibre lying on the pixels of the sensor. In such case, the counter **303** of the shadowed pixels is substituted with the circuit shown in Fig. 6 and intended to evaluate the longest shadow in each measurement cycle. In case of need, it can be applied in any of the embodiments shown in Figs. 2 to 5.

The signal **S3** from the gate **302** is led to a switch **3031** whose outputs are connected with any required number of input counters **3032a, 3032b**, ... **3032n** out of which one counter, in the shown embodiment the first input counter **3032a,** is connected to a module **3033** for the selection of the maximum while the other input counters **3032b**, ... **3032n** are connected both to the module **3033** for the selection of the maximum and to a detector **3034** of plurality of shadows. The signal **S3** from the gate **302** is at the same time led into a detector **3036** of shadow edge whose output is led into the switch **3031.** The output of the module **3033** for the selection of the maximum is led into a terminal counter **3035** whose output is intended to be connected to the divider **309.** The terminal counter **3035,** the module **3033** for the selection of the maximum, the detector **3034** of plurality of shadows, and the input counters **3032a**, **3032b**, ... **3032n** are connected with the resetting output of the control module **305.** The detector **3034** of plurality of shadows is fitted with a lug intended for the connection with the module **304** of the data bus to which it transmits information on a measuring fault on the sensor **1** such as due to dust particles on one or more pixels **11**.

During one measurement cycle, for instance with the embodiment shown in Fig. 2a, the signal **S3** of the number of shadowed pixels is led into the switch **3031** and into the detector **3036** of the shadow edge which upon the detection of the initial edge of the shadow instructs the switch **3031** to connect the first input counter **3032a.** Upon the detection of the end edge of the shadow, the detector **3036** instructs the switch **3031** to disconnect the first input counter **3032a.** Upon the detection of another initial edge of the shadow the detector **3036** instructs the switch **3031** to connect the second input counter **3032b** and upon the detection of the end edge of the shadow, the detector **3036** instructs the switch **3031** to disconnect the second input counter **3032b.** Analogically connected and disconnected are the subsequent input counters **3032c** to **3032n** upon the detection of further shadow edges. Information on the extent of the shadows is transmitted from the input counters **3032a**, **3032b**, .... **3032n** into the module **3033** for the selection of the maximum in which the maximum value of the shadow length is selected and the information on it is transmitted to the terminal counter **3035** from which it is transmitted to the divider **309** depending on the kind of the connection either after each measurement cycle or after a constant or variable number of the measuring cycles, or continuously. If the second or further input counter **3032b** to **3032n** is used during one measurement cycle, information on the second or on the following shadow is transmitted to the detector **3034** of plurality of shapes, and from it to the data bus module **304.**

The means described in the preceding text for monitoring more than one shadow during one measurement cycle and for the selection of at least the maximum shadow are given by way of example and can be substituted with another connection whose principle will be the monitoring of more than one shadow on the optical line sensor **1** and the selection of the maximum length, or length of interval, of the shade.

### List of Reference Characters

- 1: optical line sensor
- 11: pixel of sensor
- 12: output of sensor
- 13: input of sensor
- 3: circuits for processing and/or evaluating the signals of the optical line sensor
- 301: comparator
- 302: gate
- 303: counter of shadowed pixels
- 304: module of data bus
- 305: control module
- 306: detector of shadow edge
- 308: auxiliary counter
- 309: divider
- 310: counter of the number of measurement cycles
- 311: decision taking module
- 3031: switch
- 3032: input counter
- 3033: module for the selection of the maximum
- 3034: detector of plurality of shadows
- 3035: terminal counter
- 3036: detector of shadow edge
- 4: custom circuit ASIC
- 5: case
- 6: data bus
- 7: processor
- S1: analog signal of sensor
- S2: signal on sensor range
- S3: signal on number of shadowed pixels
- SN: zero-setting signal
- N: number of measurement cycle
- Y: varying number of measurement cycles
- X: number of shadowed pixels

## Claims

1. A device for contactless measurement of a diameter of a moving yarn or of a yarn-like textile formation comprising an optical line sensor (1) with a plurality of pixels arranged next to each other in at least one row, which is integrated into one semiconductor integrated custom ASIC circuit (4) together with a control module (305) for timing pulse rate generation and a comparator (301) for processing and/or for evaluating the signal of the optical line sensor (1) **characterized in that** the semiconductor integrated custom ASIC circuit further comprises a counter (303) of shadowed pixels connected via a gate (302) towards the output of the comparator (301), to which output is connected a module (304) of a data bus connectable to a data bus (6) for communication with, a control and/or evaluation processor (7).

2. A device as claimed in Claim 1, **characterized in that** the comparator output (301) is connected to the detector of shadow edge, which output is connected with the input of an auxiliary counter (308), which is at the same time connected with a timing output and a reset output of the control module (305) for shadow edge of the yarn position evaluation on the optical sensor (1), while the output of the auxiliary counter (308) is connected with the input of the module (304) of a data bus.

3. A device as claimed in Claim 1 or 2, **characterized in that** a divider (309) is inserted between the counter (303) of shadowed pixels and the module (304) of the data bus (6), while a counter (310) of a number of measurement cycles is inserted between the divider (309) and the control module (305) and a decision taking module (311) is inserted between divider (309), control module (305) and the counter (310) of a number of measurement cycles, while the resetting output of the control module (305) is connected to the resetting input of the counter (303) of the shadowed pixels and to the resetting input of a counter (310) of the number of the measurement cycles.

4. A device as claimed in Claim 3, **characterized in that** the decision taking module (311) contains information about a constant number (N) of measurement cycles for determining the mean value of yarn diameter from a constant number of measurements.

5. A device as claimed in Claim 3, **characterized in that** the decision taking module (311) comprises means for determining a variable number (Y) of measurement cycles for determining the mean value of yarn diameter from a variable number of measurements.

6. A device as claimed in Claims 1 or 2, **characterized in that** a divider (309) is inserted between the counter (303) of shadowed pixels and the module (304) of the data bus (6), while a counter (310) of a number of measurement cycles is inserted between the divider (309) and the control module (305), while outputs of the control module (305) are interconnected with the counter (303) of the shadowed pixels, with the divider (309) and with the counter (310) of the number of the measurement cycles, while outputs of the counter (310) of the number of the measurement cycles are interconnected with the divider (309).

7. A device as claimed in any of Claims 1 to 6, **characterized in that** the the counter (303) of shadowed pixels is created as a circuit constructed by to the output of the gate connected switch (3031) and the detector (3036) of shadow edge, which output is connected with the input of the switch (3031), while to the outputs of the switch (3031) is connected an optional number of input counters (3032a, 3032b, ... 3032n), from which one is connected to the module for the selection of the maximum and the others are connected to the module (3033) for the selection of the maximum and at the same time to the detector (3034) of plurality of shadows, while the module (3033) for the selection of the maximum output is connected with the divider (309) input, a terminal counter (3035), the module (3033) for the selection of the maximum, the detector (3034) of plurality of shadows and the input counters (3032a, 3032b, ... 3032n) are connected with the resetting output of the control module (305) and the detector (3034) of plurality of shadows is fitted with an output connected to the module (304) of the data bus for monitoring more than one shadow during one measurement cycle and for the selection of at least the maximum shadow .

## Patentansprüche

1. Einrichtung zur berührungslosen Messung des Querschnittes des sich bewegenden Garns oder eines einem Garn ähnlichen Textilgebildes, die einen optischen Zeilensensor (1) mit einer Vielzahl der mindestens in einer Reihe nebeneinander angeordneten Pixels aufweist, der in einen integrierten kundenspezifischen ASIC-Halbleiterschaltkreis (4) integriert ist, gemeinsam mit einem Steuermodul (305) zur Bildung eines Teilertakts und mit einem Komparator (301) zur Verarbeitung und/oder Auswertung des Signals des optischen Zeilensensors (1), **dadurch gekennzeichnet, dass** der integrierte kundenspezifische ASIC-Halbleiterschaltkreis ferner einen Zähler (303) der abgeschirmten Pixels aufweist, der über ein Gate (302) an den Ausgang des Komparators (301) angeschlossen ist, an dessen Ausgang ein Modul (304) der Datensammelschiene angeschlossen ist, das an die Datensammelschiene (6) zur Kommunikation mit dem Steuer- und/oder Auswerteprozessor (7) anschließbar ist.

2. Einrichtung nach dem Anspruch 1, **dadurch gekennzeichnet, dass** der Ausgang des Komparators (301) an den Detektor der Schattenkante angeschlossen ist, dessen Ausgang mit dem Eingang des Hilfszählers (308) verkoppelt ist, der zugleich mit dem Taktausgang und dem Nullungsausgang des Steuermoduls (305) zur Ermittlung der Lage des Anfangs des Garnschattens auf dem optischen Sensor (1) verkoppelt ist, wobei der Ausgang des Hilfszählers mit dem Eingang des Moduls (304) der Datensammelschiene verkoppelt ist.

3. Einrichtung nach dem Anspruch 1 oder 2, **dadurch gekennzeichnet, dass** die Dividiereinrichtung (309) zwischen den Zähler (303) der abgeschirmten Pixels und das Modul (304) der Datensammelschiene eingelegt ist, wobei der Zähler (310) der Zahl der Messzyklen zwischen die Dividiereinrichtung (309) und das Steuermodul (305) eingelegt ist und das Entscheidungsmodul (311) zwischen Dividiereinrichtung (309), Steuermodul (305) und Zähler (310) der Zahl der Messzyklen eingelegt ist, wobei der Nullungsausgang des Steuermoduls (305) mit dem Nullungseingang des Zählers (303) der abgeschirmten Pixels und dem Nullungseingang des Zählers (310) der Zahl der Messzyklen verkoppelt ist.

4. Einrichtung nach dem Anspruch 3, **dadurch gekennzeichnet, dass** das Entscheidungsmodul (311) die Information über die konstante Zahl (N) der Messzyklen zur Ermittlung des Mittelwertes des Garnquerschnittes aus der konstanten Zahl der Messungen enthält.

5. Einrichtung nach dem Anspruch 3, **dadurch gekennzeichnet, dass** das Entscheidungsmodul (311) die Mittel zur Bestimmung einer variablen Zahl (Y) der Messzyklen zur Ermittlung des Mittelwertes des Garnquerschnittes aus der variablen Zahl der Messungen aufweist.

6. Einrichtung nach dem Anspruch 1 oder 2, **dadurch gekennzeichnet, dass** die Dividiereinrichtung (309) zwischen den Zähler (303) der abgeschirmten Pixels und das Modul (304) der Datensammelschiene eingelegt ist, wobei der Zähler (310) der Zahl der Messzyklen zwischen die Dividiereinrichtung (309) und das Steuermodul (305) eingelegt ist, wobei die Ausgänge des Steuermoduls (305) mit dem Zähler (303) der abgeschirmten Pixels, mit der Dividiereinrichtung (309) und dem Zähler (310) der Zahl der Messzyklen verkoppelt sind, wobei die Ausgänge des Zählers (310) der Zahl der Messzyklen mit der Dividiereinrichtung (309) verkoppelt sind.

7. Einrichtung nach einem der Ansprüche 1 bis 6, **dadurch gekennzeichnet, dass** der Zähler (303) der abgeschirmten Pixels als ein Schaltkreis gebildet ist, der durch einen an den Gateausgang angeschlossenen Umschalter (3031) und Detektor (3036) der Schattenkante gebildet wird, dessen Ausgang mit dem Eingang des Umschalters (3031) verkoppelt ist, wobei an die Ausgänge des Umschalters (3031) eine wählbare Vielzahl der Eingangszähler (3032a, 3032b, ... 3032n) angeschlossen ist, von denen einer ans Modul der Maximumwahl angeschlossen ist und die restlichen ans Modul (3033) der Maximumwahl und zugleich an den Detektor (3034) von mehreren Schatten angeschlossen sind, wobei der Ausgang des Moduls (3033) der Maximumwahl mit dem Eingang der Dividiereinrichtung (309) verkoppelt ist, Endzähler (3035), Modul (3033) der Maximumwahl, Detektor (3034) von mehreren Schatten und Eingangszähler (3032a, 3032b, ...3032n) sind mit dem Nullungsausgang des Steuermoduls (305) verkoppelt und der Detektor (3034) von mehreren Schatten weist einen Ausgang auf, der ans Modul (304) der Datensammelschiene zur Verfolgung von mehreren als einem Schatten während eines Messzyklus und zur Auswahl von mindestens einem Maximalschatten angeschlossen ist.

## Revendications

1. Le dispositif pour le mesurage sans contact du diamètre du fil mouvant ou du fil de la formation textile similaire, comprenant le lecteur optique linéaire (1) avec une quantité de pixels rangés au moins dans un rang côte à côte, intégré en un seul circuit ASIC semi-conducteur de clientèle intégré (4) avec le module de commande (305) pour la création de la fréquence de cadence et avec le comparateur (301) pour l'élaboration et/ou pour la sélection du signal de lecteur optique linéaire (1), **caractérisé en ce que** le circuit ASIC semi-conducteur de clientèle intégré ensuite comprend le compteur (303) des pixels offusqués connecté à travers le circuit de porte (302) vers la sortie du comparateur (301), à la sortie duquel est connecté le module (304) de la barre collectrice des données remorquable à la barre collectrice des données (6) pour la communication avec le processeur de commande et/ou de sélection (7).

2. Le dispositif suivant la revendication 1, **caractérisé en ce que** la sortie du comparateur (301) est connectée au détecteur du bord de l'ombre, la sortie duquel est couplée avec l'entrée du compteur auxiliaire (308), qui est en même temps couplé avec la sortie de la phase et la sortie zéro du module de commande (305) pour assurer la position du début de l'ombre du filé sur le lecteur optique (1), en temps que la sortie du compteur auxiliaire est couplée avec l'entrée du module (304) de la barre collectrice des données.

3. Le disposotif suivant la revendication 1 ou 2, **caractérisé en ce que** le diviseur (309) est inséré entre le compteur (303) des pixels offusqués et le module (304) de la barre collectrice des données, en temps que le compteur (310) des nombres de cycles de mesurage est inséré entre le diviseur (309) et le module de commande (305), et le module décisionnel (311) est inséré entre le diviseur (309), le module de commande (305) et le compteur (310) des nombres de cycles de mesurage, en temps que la sortie zéro du module de commande (305) est couplée avec l'entrée zéro du compteur (303) des pixels offusqués et avec l'entrée zéro du compteur (310) des nombres de cycles de mesurage.

4. Le disposotif suivant la revendication 3, **caractérisé en ce que** le module décisionnel (311) comprend l'information du nombre constant (N) des cycles de mesurage pour le dépistage de la valeur moyenne du diamètre de filé du nombre constant de mesurage.

5. Le disposotif suivant la revendication 3, **caractérisé en ce que** le module décisionnel (311) comprend les moyens pour la détermination du nombre variable (Y) des cycles de mesurage pour le dépistage de la valeur moyenne du diamètre de filé du nombre variable de mesurage.

6. Le disposotif suivant la revendication 1 ou 2, **caractérisé en ce que** le diviseur (309) est inséré entre le compteur (303) des pixels offusqués et le module (304) de la barre collectrice des données, en temps que le compteur (310) des nombres de cycles de mesurage est inséré entre le diviseur (309) et le module de commande (305), en temps que les sorties du module de commande (305) sont couplées avec le compteur (303) des pixels offusqués, le diviseur (309) et le compteur (310) des nombres de cycles de mesurage, en temps que les sorties du compteur (310) des nombres de cycles de mesurage sont couplées avec le diviseur (309).

7. Le dispositif suivant quelconque des revendications de 1 à 6, **caractérisé en ce que** le compteur (303) des pixels offusqués est créé comme le circuit formé pour la sortie du circuit de porteconnectée par le commutateur (3031) des pixels offusqués et le détecteur (3036) du bord de l'ombre, la sortie duquel est couplée avec l'entrée du commutateur (3031), en temps qu'aux sorties du commutateur (3031) est connectée une quantité éligible des compteurs d'entrée (3032a, 3032b, ...3032n), dont un d'entre eux est connecté au module de sélection maximum et les autres sont connectés au module (3033) de séléction maximum et, en même temps au détecteur (3034) de plus d'ombres, en temps que la sortie du module (3033) de sélection maximum est couplée avec l'entrée du diviseur (309), le compteur final (3035), le module (3033) de sélection maximum, le détecteur (3034) de plus d'ombres et les compteurs d'entrée (3032a, 3032b, ... 3032n) sont couplés avec la sortie zéro du module de commande (305) et le détecteur (3034) de plus d'ombres est muni de la sortie qui est connectée au module (304) de la barre collectrice des données pour l'observation de plus d'une ombre pendant un cycle de mesurage et la sélection au moins d'une ombre maximale.
